(19) **Europäisches Patentamt / European Patent Office / Office européen des brevets**

(11) **EP 3 635 394 B1**

(12) **EUROPEAN PATENT SPECIFICATION**

(45) Date of publication and mention of the grant of the patent:
**09.10.2024 Bulletin 2024/41**

(21) Application number: **18736803.0**

(22) Date of filing: **23.05.2018**

(51) International Patent Classification (IPC):
**G01N 33/49** $^{(2006.01)}$

(52) Cooperative Patent Classification (CPC):
**G01N 33/492; G01N 21/8483;** G01N 21/255;
G01N 27/4145

(86) International application number:
**PCT/EP2018/063526**

(87) International publication number:
**WO 2018/215554 (29.11.2018 Gazette 2018/48)**

(54) **METABOLITE DETECTION APPARATUS AND METHOD OF DETECTING METABOLITES**

METABOLITDETEKTOR UND VERFAHREN ZUR DETEKTION VON METABOLITEN

APPAREIL DE DÉTECTION DE MÉTABOLITE ET PROCÉDÉ DE DÉTECTION DE MÉTABOLITES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.05.2017 GB 201708339**

(43) Date of publication of application:
**15.04.2020 Bulletin 2020/16**

(73) Proprietor: **The University Court Of The University Of Glasgow**
**Glasgow, Strathclyde G12 8QQ (GB)**

(72) Inventors:
- **CUMMING, David Robert Sime**
  **Glasgow, Strathclyde G12 8QQ (GB)**
- **PATIL, Samadahn**
  **Glasgow, Strathclyde G12 8QQ (GB)**
- **AL-RAWHANI, Mohammed**
  **Glasgow, Strathclyde G12 8QQ (GB)**
- **BARRETT, Michael**
  **Glasgow, Strathclyde G12 8QQ (GB)**
- **DHEEMAN, Dharmendra**
  **Glasgow, Strathclyde G12 8QQ (GB)**
- **HU, Chunxiao**
  **Glasgow, Strathclyde G12 8QQ (GB)**

(74) Representative: **Mewburn Ellis LLP**
**Aurora Building**
**Counterslip**
**Bristol BS1 6BX (GB)**

(56) References cited:
EP-A1- 2 230 314    EP-A1- 3 024 481
EP-B1- 3 024 481    WO-A1-2010/047804
US-A1- 2005 249 633    US-A1- 2006 257 958
US-A1- 2013 161 190    US-A1- 2016 080 548

- MOHAMMED A. AL-RAWHANI ET AL: "A Colorimetric CMOS-Based Platform for Rapid Total Serum Cholesterol Quantification", IEEE SENSORS JOURNAL., vol. 17, no. 2, 15 January 2017 (2017-01-15), US, pages 240 - 247, XP055492802, ISSN: 1530-437X, DOI: 10.1109/JSEN.2016.2629018
- CHEAH BOON CHONG ET AL: "An Integrated Circuit for Chip-Based Analysis of Enzyme Kinetics and Metabolite Quantification", IEEE TRANSACTIONS ON BIOMEDICAL CIRCUITS AND SYSTEMS, IEEE, US, vol. 10, no. 3, 1 June 2016 (2016-06-01), pages 721 - 730, XP011609066, ISSN: 1932-4545, [retrieved on 20160304], DOI: 10.1109/TBCAS.2015.2487603

**Description**

FIELD OF THE INVENTION

The present invention relates to a process and apparatus for point-of-care real time metabolite sensing. In particular, the invention relates to an apparatus and process that enables multiple metabolites to be detected simultaneously from a single sample.

BACKGROUND OF THE INVENTION

Metabolism is a vital cellular process, and its malfunction can be a major contributor to many diseases. Metabolites (i.e. substances involved in metabolism) can be good indicators of disease phenotype, and can serve as a metabolic disease biomarkers. Therefore quantification and analysis of metabolites can play a significant role in the study and early diagnosis (detection) of many diseases.

Metabolite biomarkers of different diseases are also becoming increasingly well understood which paves the way for developing new diagnostic systems. The importance of the link between metabolomics and a person's state of health is governing the need to look at both targeted and untargeted metabolites. A single metabolite can be a biomarker for several different diseases. In addition, multiple metabolites together can serve as a biomarker for a particular disease. It is therefore often necessary to detect and quantify the presence of multiple metabolites in order to accurately identify a disease. Metabolite biomarker profiling deals with the screening of a huge number of metabolites, and there are a particular panel of metabolite biomarkers which are good indicators of a person's state of health. In particular, multiplexed assaying, where multiple biomarkers are simultaneously measured in a single run, has the capacity to provide resource-rich information for decision making and prognosis, leading to the correct diagnosis and treatment for complex disease conditions such as stroke, cancer and cardiovascular diseases where directed therapy is important.

A commonly used technique for detecting and quantifying metabolites is mass spectrometry (MS). This involves ionising a chemical species and sorting the product ions based on their mass-to-charge ratios. Separation methods such as gas chromatography and liquid chromatography are often required prior to performing a mass spectrometry measurement. Nuclear magnetic resonance (NMR) spectroscopy is another technique which is used for metabolite studies. NMR can be used to detect, identify and quantify a wide range of metabolites without having to first separate them. However, both of these techniques require bulky and expensive equipment, which confines their use to hospitals and laboratories.

As an example, elevated cholesterol levels are well known for their association with an increased risk of coronary heart disease (angina or heart attack), narrowing of the arteries (atherosclerosis), stroke, peripheral heart disease and hypertension. Such conditions are often correlated with poor diet, an excessive fat intake, lack of exercise and other lifestyle choices. Measuring or therapeutic monitoring of cholesterol level in blood serum helps to assess susceptibility of the person to develop coronary artery diseases and hence is a good indicator of the state of health of a person. One of the diagnostic methods for quantifying cholesterol concentration depends on enzyme-based assays that require a spectrophotometer to measure changes in intensity of colour products from those enzyme reactions. A general purpose spectrophotometer would incorporate a sophisticated setup of a white light source, a monochromator containing a diffraction grating and a light transducer that converts light into electrical signal such as a charge-coupled device (CCD), a photodiode or a photomultiplier tube. The wide spectrum range of the spectrophotometer makes it bulky and power hungry which consequently confines its usefulness to laboratories and hospitals. Another method involves metabolites undergoing chemiluminescence reaction. This method requires a more specialised light detector such as a charge-coupled detector (CCD) to detect low light emission of luminol that is used for quantification of small analyte concentration.

More recently, use of a photodiode in a disposable sensing platform to measure change in colour of enzyme assay has been demonstrated as a means of detecting cholesterol by measuring intensity of transmitted light through assay solution [1]. The platform was based on a complementary metal oxide semiconductor (CMOS)-based photodiode array and an off-the-shelf light emitting diode (LED). The photodiode array is fabricated using commercial standard CMOS process, which is readily available for low-cost mass-production.

Photodiodes made in a CMOS process are generally sensitive to light in the 200 nm to 1100 nm range, owing to the bandgap of silicon (1.12 eV). This range makes them suitable for colorimetric enzyme assays that use visible light or fluorescent mediators, which often use wavelengths in the range 400 nm to 700 nm. A colour change within this range can be exploited for a range of enzyme assays, e.g. cholesterol ester hydrolase, cholesterol dehydrogenase, cholesterol esterase and cholesterol oxidase can be exploited to measure metabolites such as cholesterol. For metabolites with low concentrations, more sensitive CMOS compatible detector such as single photon avalanche diode (SPAD) can be integrated on the same chip and therefore, increase the dynamic detection range.

In other recent work, another type of CMOS-based chip fabricated with an integrated ion-sensitive field effect transistor (ISFET) array was used to measure glucose concentration in blood through the activity of hexokinase. The action of the hexokinase on the glucose releases hydrogen ions that are detected by the ISFET [2].

Point of care diagnostics are transforming the healthcare industry, by facilitating the use of home-testing to provide an early indication of potential illness and disease. The development of low-cost, rapid, specific and high sensitivity consumable biosensors are at the forefront of the research for user-orientated testing, driven in part by the need for rapid diagnosis and monitoring without overburdening the resources of the healthcare services. For example, glucose biosensors have become widespread in their use for managing diabetes. Liquid delivery is crucial for point of care (i.e. portable) diagnostic devices. A paper strip (e.g. chromatography paper or nitrocellulose membrane) has been proven to be an effective approach for delivering liquid samples. One well known example is the pregnancy test which measures the level of human chorionic gonadotropin (HCG) in human blood or urine to give an indication of pregnancy status.

US 2016/0080548 discloses a smartphone-based technique for imaging a colorimetric reaction of a target sample on a test strip.

WO 2010/047804 discloses an apparatus comprising an array of sample-retaining regions capable of retaining a chemical or biological sample. Each sample-retaining region may have a chemical field transistor associated with it and configured to generate at least one output signal related to a characteristic of the sample.

US 2005/0249633 A1 discloses analytical systems, devices, and cartridges therefor, for detecting or quantifying at least two different analytes using at least two different techniques, in a single sample. The cartridges typically comprise at least two test sites and the location of at least one test site is not dependent on a corresponding measurement device. The systems generally comprise a device, memory, and a processing module. The device comprises a light source, an array detector, and a port configured to accept at least a portion of a cartridge. The processing module is configured to perform an image analysis of the cartridge. The methods comprise the steps of acquiring calibration information, acquiring an image of the cartridge, performing an image analysis, and cycling through specific detection or quantification techniques corresponding to the techniques required by the test sites. Computer readable media are also described.

US 2013/0161190 A1 discloses an integrated test device for optical and electrochemical assays and, more particularly, test devices having the ability to perform optical and electrochemical assays and methods of performing optical and electrochemical assays using such test devices. Such devices may be particularly useful for performing immunoassays and/or electrochemical assays as the point-of-care.

EP 2 230 314 A1 discloses a method of sensing an analyte that comprises: passing a fluid over a sensor array, the sensor array comprising at least one particle positioned within a cavity of a supporting member, wherein the particle comprises a polymeric resin and a receptor coupled to the polymeric resin; monitoring a spectroscopic change of the particles as the fluid is passed over the sensor array, wherein the spectroscopic change is caused by the interaction of the analyte with the particle. The receptor can be an enzyme substrate and the analyte an enzyme such as protease, nuclease or glycosidase.

## SUMMARY OF THE INVENTION

At its most general, the present invention provides a CMOS-based chip having one or more sensing modalities that are able independently to detect multiple biomarkers (e.g. metabolites that acts as metabolic biomarkers) present in a sample. In particular, the invention relates to a scenario in which detection by the multiple sensing modalities occurs at different locations with respect to the chip, whereby the chip can simultaneously detect a plurality of metabolites by measuring behaviour of a test material in the different locations. With this technique, multiple metabolites may be measured in real time using a small scale point-of-care device.

According to first and second aspects of the invention, there is provided an apparatus for detecting biomarkers in a biological sample as set out in claims 1 and 5 respectively. The apparatus comprises: a sample receiving module arranged to receive the biological sample and transport it to a reaction zone for testing, wherein the reaction zone comprises a first testing region and a second testing region spatially separated from the first testing region, wherein properties of the first testing region and the second testing region are affected by the presence of metabolites to be detected; and a CMOS-based sensor unit disposed in relation to the reaction zone to detect independently the properties of the first testing region and the second testing region thereby to obtain separate signals indicative of the presence of metabolites in each of the first testing region and the second testing region. By probing different regions of a reaction zone, the invention effectively provides a multiplexed measurement system, where separate signals corresponding to different metabolites can be obtained from the same device. This is particularly useful where the first and second testing regions detected by the sensor unit are independently affected by one or more metabolites to be detected, i.e. there is substantially no cross-talk between the signals.

Herein the phrase "CMOS-based" may mean that the device is capable of fabrication using conventional semiconductor chip processes, e.g. comprising a series of depositing, masking and etching steps on a substrate. The sensor unit and its constituent components may thus be semiconductor components. This may enable the sensor unit to be mass-produced at low cost. The apparatus may thus be embodied as a compact hand-held device which is easily transportable, thus facilitating rapid point-of-care diagnostics. Compared with current analytical methods for metabolite detection and quantification, no expensive detection equipment is required.

The sensor unit itself may resemble a semiconductor chip, and may have mounted thereon or connectable thereto means for controlling and processing the chip functions. For example, the apparatus may comprise a controller, e.g. a microprocessor or the like, arranged to send and receive signals from the sensor unit. For example, the controller may be arranged to activate the sensor unit by applying an appropriate voltage.

The properties of the first testing region and the second testing region that are detected by the sensor unit may be physical or chemical. For example, the sensor unit may be arranged to detect changes in appearance, chemical composition, mass, temperature, etc.

The reaction zone may have more than two discrete testing regions. For example, there may be three, four, five or more testing regions, as space allows. References to the first and second testing region below should be understood as being equally relevant to examples with more than two testing regions.

In the first aspect of the invention, the first testing region and the second testing region are sensitive to different metabolites. That is, the first testing region is sensitive to a first metabolite, and the second testing region is sensitive to a second metabolite, whereby the separate signals are indicative of the presence of the first metabolite and second metabolite respectively. The reaction zone may include a control region that is not sensitive to the presence of metabolites to be detected, e.g. to provide a reference signal against which signals from the first testing region and second testing region can be compared.

The first testing region and the second testing region may be physically separated to prevent cross-talk therebetween. For example, a fluid flow barrier may separate the first testing region from the second testing region to inhibit or prevent transfer of the biological sample therebetween.

In the second aspect of the invention, the first testing region and the second testing region each comprise a respective micro-well, the micro-wells being separated from each other by a barrier portion. The barrier portion may be a raised part of the reaction zone between the micro-wells. It may be shallow, e.g. having a height relative to the base of the micro-wells in the range 1 to 200 $\mu$m, preferably 1 to 100 $\mu$m. The reaction zone may include four or more micro-wells. The micro-wells may be pre-loaded with an enzyme (and/or other reagents) in preparation to react with a substance in a sample.

The first testing region and/or the second testing region may comprise a test material arranged to support a metabolite-activated reaction upon receiving the biological sample. For example, each of the first testing region and/or the second testing region may each comprise an assay region, e.g. located within a microfluidic channel. The microfluidic channel may be pre-loaded with a test solution comprising one or more enzymes. Properties of the test solution may change due to a reaction between the enzymes and metabolites to be detected.

The biological sample is typically a liquid. In one example, the sample receiving module comprises a paper strip or other capillary structure for transporting the liquid biological sample to the reaction zone, e.g. by capillary action. Any suitable material that exhibit a wicking ability may be used for the paper strip. For example, chromatography paper or nitrocellulose membrane can be used. The paper strip may be disposed over the CMOS-based sensor unit, i.e. to carry the biological sample directly into or over the reaction zone.

In one example, the reaction zone includes microfluidic channels arranged to draw the biological sample away from the paper strip. In this case, the first testing region and the second testing region may be disposed on the CMOS-based sensor unit. However, in other examples the first testing region and the second testing region are integrally formed in the paper strip. This may enable the sensor unit to be used for different combinations of testing regions, and may enable the sensor unit to be used repeatedly without needing to clean or re-load the reaction zone with a test solution.

The CMOS-based sensor unit may comprise an optical sensor. The optical sensor may be arranged to detect changes in the appearance of the reaction zone, e.g. by capturing an image or determining a change in optical properties thereof. The apparatus may comprise an optical source (e.g. LED or the like) for illuminating the test material with optical radiation. In one example, the optical sensor may be a spectral absorption sensor, e.g. a photodiode or an array of photodiodes or/and a single photon avalanche diode (SPAD) to increase the detection dynamic range.

The CMOS-based sensor unit may have multiple sensing modalities. For example, it may comprise a substrate having a first sensing element and a second sensing element fabricated thereon. The first sensing element and the second sensing element may be arranged to detect simultaneously different properties of the reaction zone to enable simultaneous detection of a plurality of metabolites. The first sensing element may comprise an optical sensor (e.g. as described above). The second sensing element may detect a different property from the first sensing element. For example, the second sensing element may be a chemical sensor, e.g. arranged to determine a change in composition or chemistry within the reaction zone (or within one or more of the testing regions). In one example, the second sensing element is a pH sensor, e.g. comprising an ion sensitive field effect transistor (ISFET) having a gate electrode in contact with the test material. The apparatus may include a reference electrode arranged to apply a voltage to the reaction zone.

The apparatus may comprise an array of CMOS-based sensor units. Respective signals can be measured from each sensor unit. Alternatively, an average signal can be measured from the array of sensor units. Measuring an average signal can greatly reduce signal noise: according to Gaussian statistics, signal noise is reduced as a function of $\sqrt{N}$, where *N* is the number of sensor units.

Each CMOS-based sensor unit in the array may be independently addressable to obtain signals corresponding to each of the testing regions.

The biological sample may be blood serum, but the invention can be used with any biological sample capable of communicating metabolites into the reaction zone.

In a further aspect, the invention may provide a method of detecting biomarkers in a biological sample as set out in claim 14.

BRIEF DESCRIPTION OF THE DRAWINGS

Embodiments of the invention will now be described by way of example with reference to the accompanying drawings, in which:

Fig. 1 shows a plan view of a CMOS chip with an array of sensors;
Fig. 2 shows a plan view of a first multiplex assay apparatus for performing multiple simultaneous measurements;
Fig. 3 shows a close up view of a CMOS chip used in the apparatus of Fig. 2;
Fig. 4 shows a close up view of a paper strip used in the apparatus of Fig. 2;
Fig. 5 shows a plan view of a second multiplex apparatus for performing multiple simultaneous measurements;
Fig. 6 shows a close up view of a paper strip used in the apparatus of Fig. 5;
Fig. 7 shows a schematic view of an ion-sensitive field-effect transistor which may be used in the present invention;
Fig. 8 shows a plan view of a third multiplex assay apparatus for performing multiple simultaneous measurements;
Fig. 9 shows a close up view of a paper strip used in the apparatus of Fig. 8;
Fig. 10 shows a schematic view of an alternative paper strip;
Fig. 11 is a set of graphs showing measurements obtained from three channels on a single paper strip;
Fig. 12 shows a schematic view of a fourth multiplex assay apparatus for performing multiple simultaneous measurements;
Fig. 13 shows a close up view of a CMOS chip used in the apparatus of Fig. 12; and
Fig. 14 is a schematic side view of an optical alignment tool for manufacturing a CMOS chip that is an embodiment of the invention.

DETAILED DESCRIPTION; FURTHER OPTIONS AND PREFERENCES

Embodiments of the present invention provide a metabolite detection device arranged to detect simultaneously multiple metabolites from a single biological sample. The device includes a reaction zone with spatially separated testing regions that have properties that are sensitive to the presence of different metabolites. The device comprises a single CMOS-based chip having one or more sensing modalities capable of detecting the properties of the separate testing region to determine the presence of multiple metabolites in the sample.

The one of more sensing modalities are provided by components fabricated on to the CMOS-based chip. In the examples discussed below, the sensing modalities include an optical sensor, e.g. for sensing optical radiation, and a pH sensor, e.g. for sensing a concentration of ionic species in a sample. However, it may be understood that the principles of the invention are applicable to any kind of sensor that can be fabricated or post processed on a CMOS chip and which is capable of detecting information indicative of the presence of a metabolite.

The sample may be a biological sample (e.g. fluid or tissue) obtained from a subject in any conventional manner. In the example discussed below the sample is blood serum, but it should be understood that the invention may encompass the use of other (or additional) sample types such as urine, sweat and swab from other body openings.

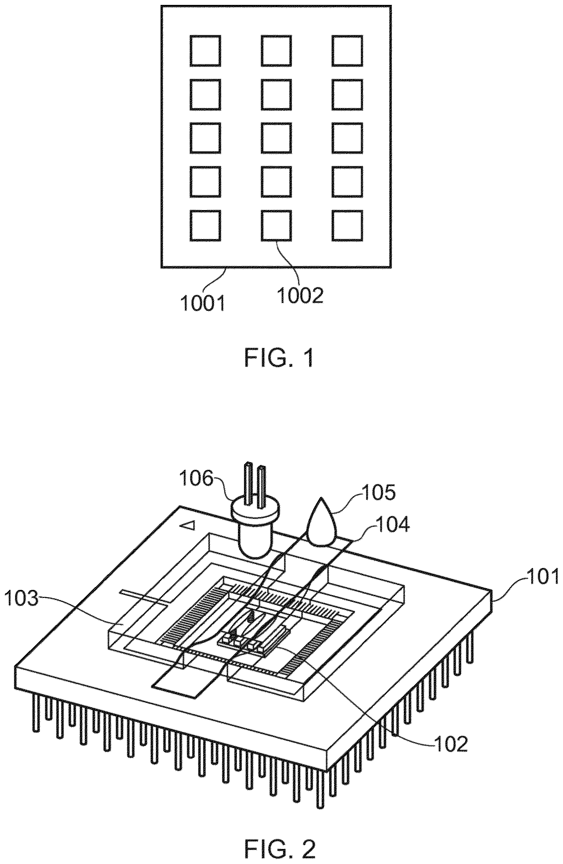

Fig. 1 shows a plan view of a complementary metal oxide semiconductor (CMOS) chip 1001 having an array of sensors 1002 across the surface of the chip. The chip 1001 is typically a silicon integrated circuit (IC), and the sensors 1002 may be photosensitive (e.g. photodiodes or/and single photon avalanche diodes (SPADs)) or chemical sensors (e.g. ion-sensitive field-effect transistors (ISFETs) or electrochemical electrodes) as will be explained in further detail below. Alternatively, in certain embodiments which are indicated below, each sensor 1002 may include a pH sensor in addition to a photodiode.

The present invention relates to the use of a single chip of the kind shown in Fig. 1 to make multiple simultaneous measurements on a liquid sample (e.g. blood, blood serum, urine) by dividing the array of sensors 1002 into multiple assay regions. Division of the sensors 1002 can be done by physical separation of the assay regions on the chip itself or by providing discrete treatment zones (e.g. multiple microfluidic channels) on a paper strip which is used to introduce a liquid sample to the sensors 1002. Although the chip 1001 shown is a $3 \times 4$ array of sensors 1002, the present invention may comprise a $16 \times 16$ array of sensors 1002.

Fig. 2 shows a schematic perspective view of a first multiplex assay apparatus for performing multiple simultaneous measurements on a liquid sample 105.

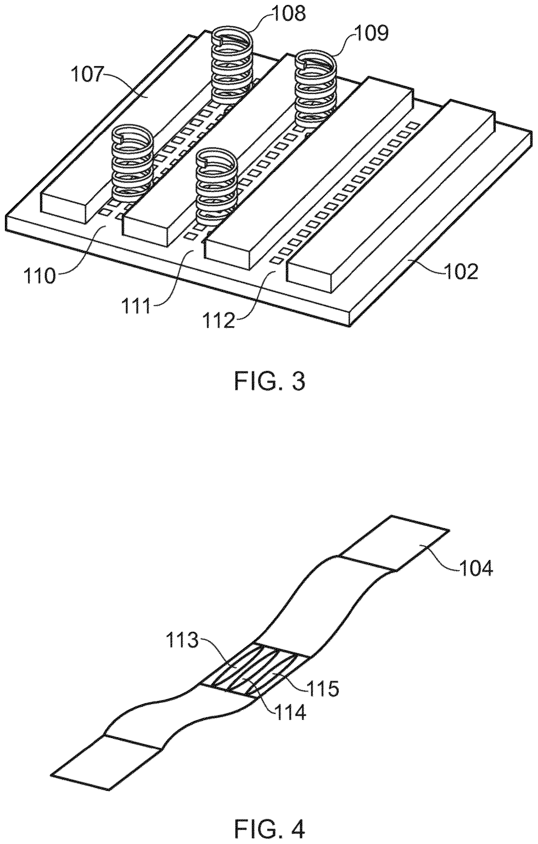

Close up views of the chip 102 and paper strip 104 used for the assay are shown in Figs. 3 and 4, respectively. The assay apparatus comprises a chip carrier 101 for a CMOS chip 102. The surface of the chip 102 has an array of photodetectors (e.g. photodiodes or/and SPADs), in the manner shown in Fig. 1. An epoxy layer 103 is provided on the chip carrier 101 to form a channel across the surface of the chip 102 for receiving a paper strip 104. The epoxy layer 103 also protects wire bindings between the chip 102 and chip carrier 101. An LED 106 is positioned above the chip 102 to illuminate the photodiodes such that they are able to detect a change in colour during the assay, as described below.

The chip 102 is shown in more detail in Fig. 3. A series of physically discrete treatment zones are provided by microfluidic channels 110, 111, 112. In this example, there are three channels. Two of the channels are activated to respond to substances to be detected, and the third channel is a control. The principles of the invention are not limited to this arrangement. There may be any number of channels activated in a manner to detect a plurality of different substances.

The channels are fabricated with a photoresist 107 on top of the chip 102, which is glued and wire-bound to the chip carrier 101. Microfluidic channel I 110 is coated with enzyme I 108; microfluidic channel II 111 is coated with enzyme II 109; and microfluidic channel III 112 is not coated with any enzyme so as to give a negative control channel. In this way, the photoresist physically separates assay regions on the chip 102 itself.

The paper strip 104 is shown in more detail in Fig. 4. The paper strip 104 is sized to fit the channel formed in the epoxy layer 103 across the surface of the chip 102. The paper strip 104 has a reaction zone that is arranged to fit over the chip in use. The reaction zone is modified using a hydrophobic polymer to form three microfluidic channels 113, 114, 115 which correspond respectively to the three microfluidic channels 110, 111, 112 on the surface of the chip 102. The polymer confines the sample that is transported along the paper strip within the three channels 113, 114, 115. This arrangement prevents cross-talking and cross-contamination.

To perform a multiplexed assay using the apparatus shown in Fig. 2, the paper strip 104 is inserted into the channel formed in the epoxy layer 103 until the reaction zone is in contact with the surface of the chip 102. A drop of analyte solution 105 is applied to one end of the paper strip 104. The analyte solution contains (possible among other things) substances I and II, which may be different metabolites, wherein enzyme I 108 is specific for substance I and enzyme II 109 is specific for substance II. Due to capillary force, the analyte solution 105 flows along the paper strip 104 to the top of the chip 102, where reactions takes place in the microfluidic channels. The enzyme reactions may generate colour changes on the paper strip 104. Enzyme I 108 generates one colour change 113, and enzyme II 109 generates a different colour change 114. The negative control channel generates no colour change 115. The colour changes are detected in real time by the photodiodes on the chip 102 under the illumination of LED 106, the multiple colour changes producing multiple detections. The change in colour is detected as light absorption by the enzyme reaction products. In this way, the apparatus allows the detection of multiple metabolites in a single assay.

In the configuration depicted in Figs. 2-4, microfluidic channels 110-112 are defined on the surface of the chip 102. Each microfluidic channel is a distinct assay region which is physically separated from the other channels and which has its own photodiodes. The paper strip 104 wets the surface of the chip 102 such that the analyte solution 105 is drawn down into the microfluidic channels 110-112 on the chip surface. Optical cross-talk is limited as the colour change chemistry is in an immediately proximal channel, the channels being physically separated by the photoresist 107. Cross-talk may also occur by chemical diffusion and capillary action from the microfluidic channels on the chip into the paper, and subsequent transfer across into an adjacent channel. Such cross-talk is minimised by ensuring that the distance between adjacent microfluidic channels is large enough, for example by thickening the walls of photoresist 107.

By replacing the photosensitive sensors with chemical sensors, the apparatus described may be suitable for detecting reactions by a pH change of the solution. This is described in more detail below.

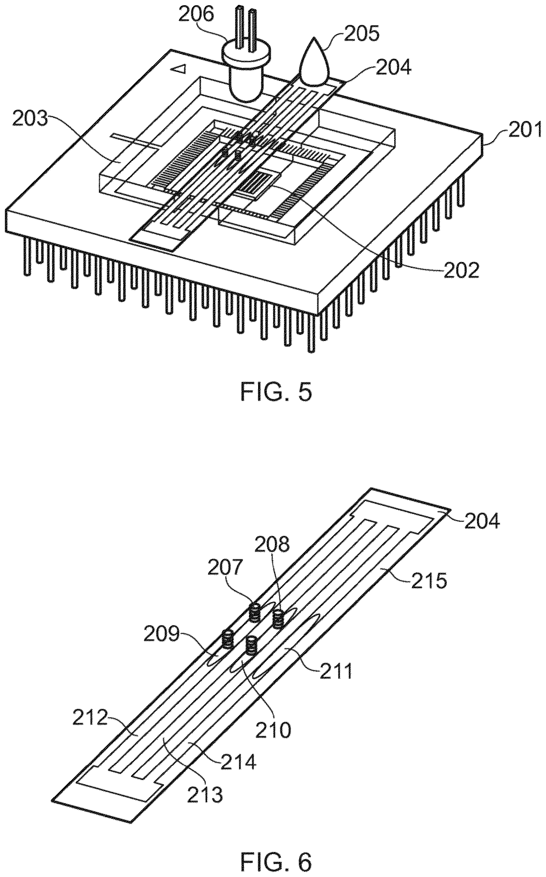

Fig. 5 shows a plan view of a second multiplex assay apparatus for performing multiple simultaneous measurements on a liquid sample 205. A close up view of the paper strip 204 is shown in Fig. 6. The assay apparatus comprises a chip carrier 201 for a CMOS chip 202. The surface of the chip 202 has an array of photodiodes and/or single photon avalanche diodes, in the manner shown in Fig. 1. An epoxy layer 203 is provided on the chip carrier 201 to form a channel across the surface of the chip 202 for receiving a paper strip 204. The epoxy layer 203 also protects wire bindings between the chip 202 and the chip carrier 201. An LED 206 having a known, specific wavelength is positioned above the chip 202 to illuminate the photodiodes such that they are able to detect a change in colour during the assay, as described below.

The paper strip 204 is shown in more detail in Fig. 6. The paper strip 204 is sized to fit the channel formed in the epoxy layer 203 across the surface of the chip 202. The paper strip is modified with a hydrophobic polymer 215 to form three microfluidic channels 212, 213, 214 which extend a substantial distance between a first end and a second end of the elongate paper strip 204. The hydrophobic polymer 215 prevents cross talk and cross-contamination between the three microfluidic channels 212, 213, 214. Microfluidic channel I 212 is coated with enzyme I 207; microfluidic channel II 213 is coated with enzyme II 208; and microfluidic channel III 214 is not coated with any enzyme so as to give a negative control channel. Different assay regions are thereby defined by the different channels on the paper strip 204.

To perform a multiplexed assay using the apparatus shown in Fig. 5, the paper strip 204 is inserted into the channel formed in the epoxy layer 203. To prevent cross talk and cross-contamination the paper strip 204 should not come into contact with the surface of the chip 202, but should instead rest a distance away from the chip 202. A drop of analyte solution 205 is applied to one end of the paper strip 204. The analyte solution contains substrates I and II, which may be different metabolites, wherein enzyme I 207 is specific for substrate I and enzyme II 208 is specific for substrate II. Due to capillary force, the analyte solution 205 flows along the paper strip 204 through the microfluidic channels 212-214, where reactions with the enzymes 207, 208 take place. The enzyme reactions produce colour changes on the paper strip 204. Enzyme I 207 generates one colour change 209 in microfluidic channel I 212, and enzyme II 208 generates another colour change 210 in microfluidic channel II 213. Microfluidic channel III 214 for negative control generates no colour change 211. The colour changes are detected in real time by the photodiodes on the chip 202 under the illumination of LED 206, the multiple colour changes producing multiple detections. In this way, the apparatus allows the detection of multiple metabolites in a single assay.

In the configuration depicted in Figs. 5 and 6, microfluidic channels 212-214 are defined on the paper strip 204. Each microfluidic channel is a distinct assay region which is physically separated from the other channels. The paper strip 204 is in close proximity to the photodiodes on the surface of the chip 202, but it is not in contact. Cross talk may occur by optical scattering of large angles through the paper casting light onto adjacent sensors. The distance between the chip 202 and the paper strip 204 is made small so as to limit or eliminate such optical cross talk. In addition, channel spacing on the paper strip 204 must be large enough to keep cross talk at a low level to allow independent measurement of the colour change occurring in each microfluidic channel. This is done by varying the thickness of the microfluidic channels and the hydrophobic polymer barriers separating them.

The embodiments described above each work by detecting a colour change, using photodiodes illuminated by an LED having a known, specific wavelength. However, for embodiments where the sample is brought into contact with the chip, the invention may alternatively or additionally make use of an array of chemical sensors on a chip. For example, the chemical sensors may be ion-sensitive field-effect transistors (ISFETs). An ISFET is a field-effect transistor in which a solution is used as the gate electrode. A change in pH (i.e. a change in concentration of $H^+$ ions) of the solution causes a current running though the ISFET to change by a measurable amount.

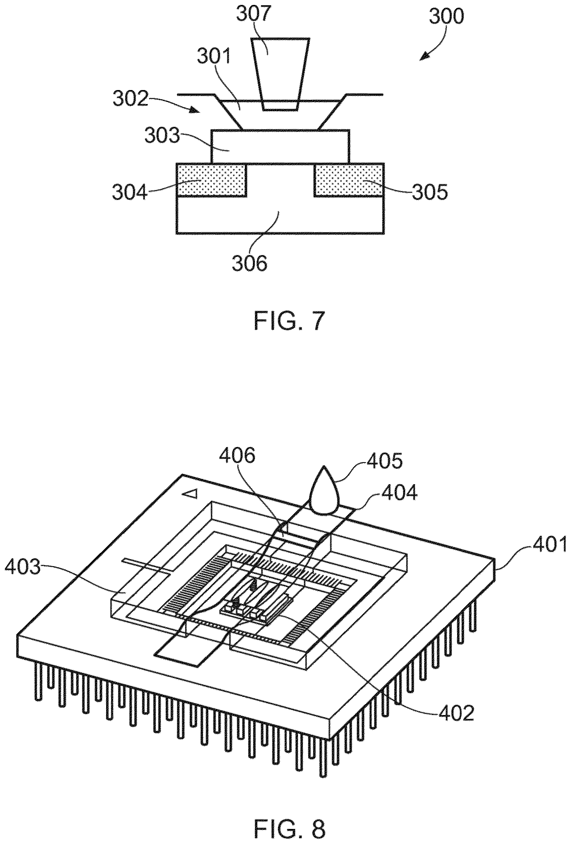

Fig. 7 shows a schematic view of an ISFET 300 which may be used as a chemical sensor for detecting the pH of a solution 301 in the present invention. The ISFET 300 comprises a well for receiving solution 301 formed by an epoxy 302 on the surface of the ISFET 300. The solution 301 contains a concentration of $H^+$ ions to be detected. The well ensures that the solution 301 is in contact with a gate oxide layer 303. A source 304 and a drain 305 are also provided in a bulk layer 306, with both source 304 and drain 305 in contact with the gate oxide 303, on a side which is opposite the solution 301. The presence of $H^+$ ions in the solution 301, which are adsorbed onto the surface of the gate oxide 303, causes migration of charge carriers to the upper surface of the bulk layer 306. Current is thereby able to flow between the source 304 and the drain 306 through the bulk layer 306. As the source/drain current is affected by the concentration of $H^+$ ions, applying a known voltage to the reference electrode 307, which is at least partially immersed in the solution 301, allows the pH of the solution 301 to be determined. Alternatively, the source/drain current can be kept constant and the voltage change at the reference electrode 307 measured to determine the pH of the solution 301.

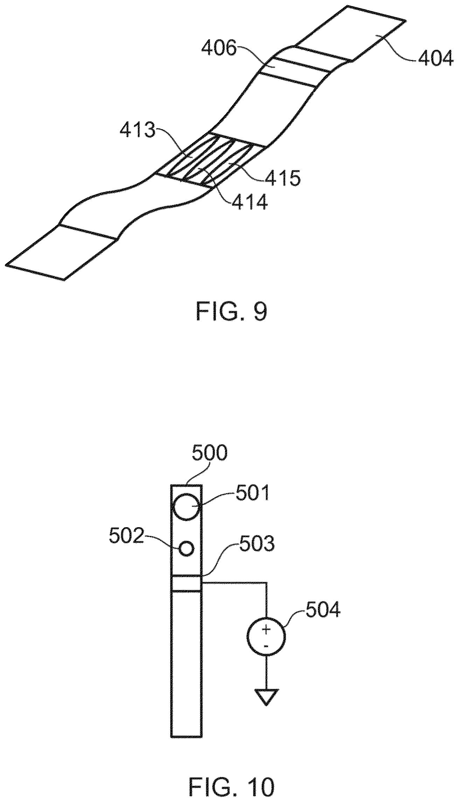

Fig. 8 shows a plan view of a third multiplex assay apparatus for performing multiple simultaneous measurements on a liquid sample 405. A close up view of the paper strip 404 used for the assay is shown in Fig. 9. The assay apparatus comprises a chip carrier 401 for a CMOS chip 402, similar to the embodiments shown above. However, in this third embodiment the surface of the chip 402 has an array of chemical sensors, in particular ISFETs, substantially as described above with reference to Fig. 7. An epoxy layer 403 is provided on the chip carrier 401 to form a channel across the surface of the chip 402 for receiving a paper strip 404. The epoxy layer 103 also protects wire bindings between the chip 402 and chip carrier 401. An LED having a known, fixed wavelength may be provided where the sensors on the chip 402 also include a photodiode, as described herein.

The chip 402 is substantially identical to chip 102 shown in Fig. 3. However, chip 402 comprises an array of chemical sensors. Chip 402 may, however, comprise a number of microfluidic channels which are coated with enzymes, and a negative control channel which is not coated with an enzyme, in the manner of chip 102 shown in Fig. 3. The microfluidic channels provide multiple distinct assay regions.

The paper strip 404 is shown in more detail in Fig. 9. The paper strip 404 is sized to fit the channel formed in the epoxy layer 403 across the surface of the chip 402. The paper strip 404 is modified with a hydrophobic polymer to form three microfluidic channels 413, 414, 415 which correspond to microfluidic channels on the surface of chip 402. This arrangement prevents cross-talking and cross-contamination. The paper strip 404 also comprises a metallised region 406, which may be printed or impregnated with silver such that the metallised region 406 may be used as a reference electrode in the assay process as described below.

To perform a multiplexed assay using the apparatus shown in Fig. 8, the paper strip 404 is inserted into the channel formed in the epoxy layer 403 until it is in contact with the surface of the chip 402. A drop of analyte solution 405 is applied to one end of the paper strip 404. The analyte solution contains multiple substrates, which may be different metabolites, wherein each enzyme in a microfluidic channel on the surface of the chip 402 is specific to a substrate, in a manner which has been described above with reference to first and second multiplex assay apparatus. Due to capillary force, the analyte solution 405 flows along the paper strip 404 to the top of the chip 402, where reactions take place in the microfluidic channels. The enzyme reactions generate pH changes on the paper strip 404; although there is no pH change in the negative control channel. The pH changes may be detected by applying a fixed reference voltage to the metallised region 406 of the paper strip 404, and measuring the change in source to drain current through each of the ISFET chemical sensors on the surface of the chip 402. To convert these current measurements to pH values, the measurements are compared with a source to drain current obtained with a solution of known pH and the same, fixed reference voltage. In this way, the apparatus allows the detection of multiple metabolites in a single assay.

Each microfluidic channel on the surface of the chip 402 is a distinct assay region which is physically separated from the other channels and which has its own chemical sensors. The paper strip 404 wets the surface of the chip 402 such that the analyte solution 405 is drawn down into the microfluidic channels on the chip surface. Cross-talk may also occur by chemical diffusion and capillary action from the microfluidic channels on the chip into the paper, and subsequent transfer across into an adjacent channel. Such cross-talk is minimised by ensuring that the distance between adjacent microfluidic channels is large enough, for example by thickening the walls of photoresist on the surface of the chip 402.

In addition to pH sensitive detectors as described, the chip 402 may comprise an array of sensors which combine a photodiode and an ISFET. In this way, the multiplex assay apparatus of Fig. 8 may also be configured to perform assays by the light absorption method described above, using an LED having a known, specific wavelength to illuminate the paper strip 404 and the chip 402. The pH change of the reactions in the microfluidic channels may also be measured at the same time as light absorption to carry out more in depth multiplex assays.

Fig. 10 shows a schematic view of an alternative paper strip 500 which may be used with the multiplex assay apparatus of Fig. 8. The paper strip 500 comprises a blister pack 501 for containing a solution, such as an electrolyte of pH buffer solution, which is able to aid transport of an analyte sample along the paper strip 500. The paper strip 500 also comprises a metallised region 503, which may be printed or impregnated with silver, to be connected to a reference voltage 504 such that the metallised region 503 can act as a reference electrode in a manner as described above.

In use, an analyte sample may be spotted at 502. The blister pack 501 can be squeezed or otherwise burst to release the solution within, and so aid transport of the analyte sample along the paper strip 500 for a multiplex assay.

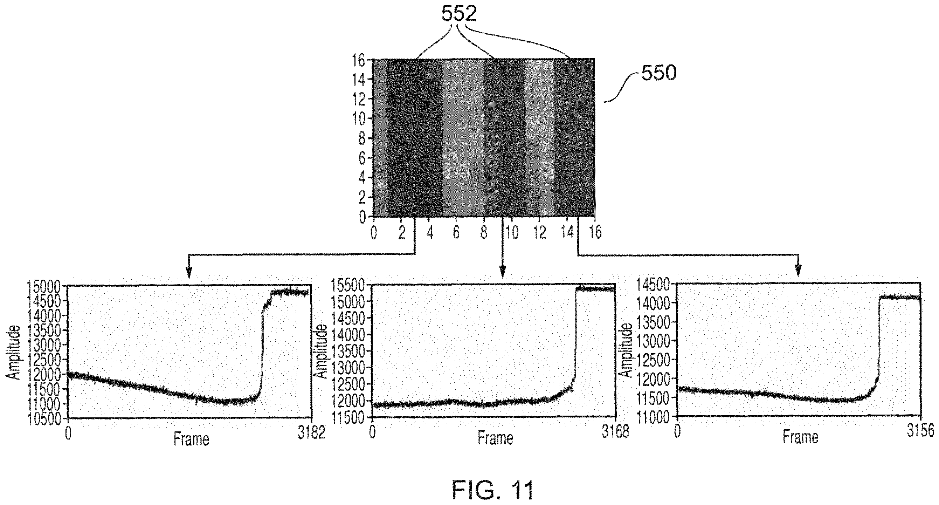

Fig. 11 shows a set of graphs that illustrate the results of a test using a single paper strip having three channels on one single paper strip. The three channels were tested simultaneously by adding one drop of analyte from one side of the paper strip. The top image 550 in Fig. 11 is an intensity graph of the three channels (dark regions 552) on the photodiode under the illumination of an LED. The middle channel was used as control (i.e. was not printed with an enzyme). The side channels were each printed with a respective enzyme (Enzyme 1 and 2). The three lower images are graphs showing electrical signal amplitude from each of the channels with time. When the analyte reached the sensing region, there is a big step jump of the electrical signal in all channels due to the wetting of the paper. After around half minute, a decreasing signal can be seen from the side channels with enzymes. As a control, the middle channel gives nearly no signal change.

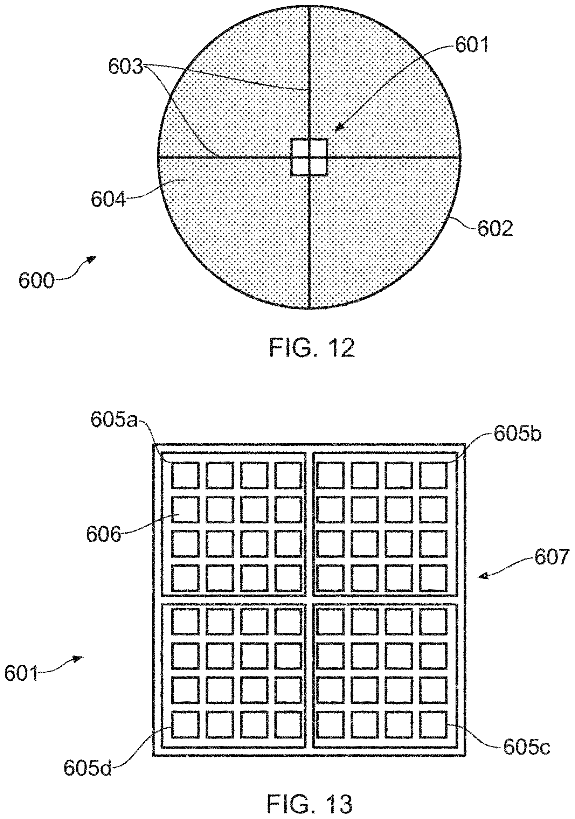

Fig. 12 shows a schematic view of a fourth multiplex apparatus 600 for performing multiple simultaneous measurements on a liquid sample. A close up view of the chip 601 used for the assay is shown in Fig. 13. The assay apparatus comprises a chip carrier (not shown) for a CMOS chip 601. Spaced away from the chip is an annular wall 602 which defines the outer edge of four reaction chambers, or quadrants, which are separated from each other by separation walls 603. A different reaction may take place in each quadrant, as explained in more detail below. An epoxy layer 604 forms the base of the reaction chambers, and also protects wire bindings between the chip 601 and the chip carrier. However, the epoxy layer 604 does not extend over the surface of the chip 601, which ensures that the surface of the chip 601 is open to be able to detect reaction parameters, such as a colour or pH change. An LED having a known, specific wavelength may be positioned above the chip 601 if the reactions are to be detected by light absorption.

The chip 601 is positioned at the meeting point between the separation walls 603 such that the chip 601 is divided into four distinct assay regions 605a, 605b, 605c, 605d. Each assay region 605 is defined by a micro-well in the epoxy layer 604. When a liquid sample is deposited in each of the reaction chambers, the micro-wells are filled such that liquid is in contact with sensors 606 in each of the assay regions 605. The assay regions 605 are separated from each other by sidewalls 607, so that liquid cannot leak into an adjacent assay region. The sensors 606 may be photosensitive (e.g. photodiodes or single photon avalanche diodes) or chemical sensors (e.g. ISFETs or electrochemical electrodes). Alternatively, the chip 601 may comprise an array of sensors 606 which combine a photodiode and an ISFET. In this way, the multiplex assay apparatus may also be configured to perform assays by the light absorption method described above, using an LED having a known, specific wavelength to illuminate the reaction chambers and the chip 601. The pH change of the reactions in the microfluidic channels may also be measured at the same time as light absorption to carry out more in depth multiplex assays.

In one example, the multiplex apparatus 600 is manufactured as follows. Each assay region 605 is protected by a polydimethylsiloxane (PDMS) block, which helps to shape an epoxy (preferably black epoxy), which is introduced in a succeeding step, and also protect the sensor array area of the chip 601 from damage during the manufacturing process. The use of black epoxy can prevent cross talk in a signal (either optical or electronic) from occurring between different assay regions (also referred to herein as micro-wells).

The PDMS blocks are positioned by microscope assisted translation on top of the chip 601. The gap between them determines the separation distance between adjacent micro-wells, and the height of the blocks is an upper limit to the micro-well depth. Epoxy mixture is then carefully poured around the blocks, and over the surface of the chip carrier to form the epoxy layer 604. The epoxy is left to cure and harden for around 12 hours. The annular wall 602 is then introduced to form the outer wall of the four reaction chambers. The annular wall 602 may be a ring made of plastics material, having a height of around 8 mm. The annular wall may be held in place with epoxy. PDMS blocks are then used to define the shape of the reaction chambers and separation walls 603. They may be shaped and positioned manually, or the separation walls may be first made from a polystyrene sheets with the desired separation wall dimensions and PDMS poured into the cavities defined by the polystyrene and annular wall 602 to be cured. When the polystyrene sheets are removed, the sidewalls 603 can be properly formed from epoxy by filling the gaps between the PDMS blocks. After curing of the epoxy, the PDMS blocks may be removed, as well as the PDMS blocks defining the micro-wells 605. The micro-wells thus have pipettable access and sidewalls 603 separate the liquid into individual reaction chambers to prevent mixing. This multilevel sequential die casting technique is compatible with CMOS processing since it can be used at room temperature. It may be expedited by raising temperature to 70°C.

In this technique, the resulting micro-wells may exhibit a step profile opening into a wider area. This allows pipettable access to the micro-wells without requiring micro-tubing or fluid management pumps for sample delivery. In turn this can save on time required for the delivery of samples.

In another example, the reaction chambers (and micro-wells discussed below) are manufactured by mounting a preformed cartridge over the chip carrier. The cartridge may be formed from any suitable material, e.g. plastic. It may have a form similar to the annular wall 602 and separation walls 603 discussed above. The cartridge may be affixed to the chip carrier in any conventional manner, e.g. by screws or the like.

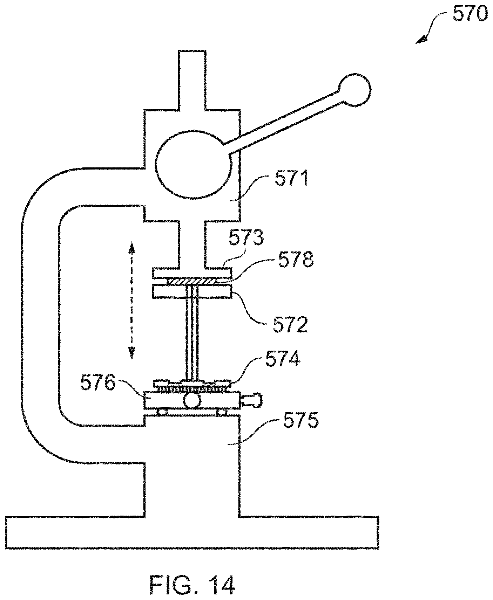

Fig. 14 is a schematic view of an optical alignment tool 570 may be used to align the cartridge 572 with the chip carrier 574 during the mounting process and to maintain alignment during the attachment (e.g. screwing) process. The optical alignment tool comprises a reciprocating movement mechanism 571 for bringing a movable holder 573 into contact with a platform 575. The chip carrier 574 is mounted on the platform 575 via an alignment stage 576 that permits adjustment in two orthogonal linear dimensions (e.g. x- and y-dimensions) in the plane of the platform 575 and a rotation dimension (e.g. about an axis extending perpendicular to the platform 575). A pivoting lever may be used to operate the linear motion of the reciprocating movement mechanism 571.

A laser source 578 is mounted on the movable holder 573 to emit a laser beam towards the platform 575. The cartridge 572 is mounted on the holder to partially block the laser beam, whereby a pattern of the separation walls is projected on to the chip carrier 574 on the platform 575.

With this arrangement, the laser illuminates a spot on the chip with a pattern that is indicative of alignment with the cartridge.

The movable holder 573 may use suction or a magnetic retainer to hold the combination of laser source and cartridge. The alignment between the cartridge and chip carrier can be adjusted via the alignment stage 576 during linear movement of the cartridge and subsequent fixing thereof to the chip carrier.

In one example, the optical alignment tool may be fabricated as part of a microscope. Visual inspection of alignment with the chip itself can be carried out through the microscope while fixing the cartridge in place.

To perform a multiplexed assay using the apparatus shown in Fig. 12, a different reaction enzyme is pipetted into each of three micro-wells 605a, 605b, 605c, leaving one micro-well 605d without an enzyme to act as a negative control. An analyte solution may then be introduced into each of the four reaction chambers. The analyte solution may contain at least three substrates, or metabolites, wherein each enzyme is specific to a substrate, as described above with reference to other embodiments of the invention. Reactions between the enzymes and substrates take place in each of the micro-wells 605a-c, where they are detected by sensors 606. For example, the reaction may cause a colour change which may be detected by photodiodes and/or a pH change which may be detected by chemical sensors in a manner substantially as described above. In this way, the apparatus allows the detection of multiple metabolites in a single assay, using a single chip 601.

In embodiments of the invention that use the micro-well arrangement discussed above, it may be desirable to have a minimum of $3\times3$ pixels per micro-well. The width of the wall separating adjacent micro-wells may be around 40 um. This ensures that the separation between micro-wells consumes no more than one pixel row or column. The walls may seal against the sensor array area of the chip using a pressure sensitive adhesive. The adhesive may expand under applied pressure, so a thickness of the walls is set within a tolerance to ensure this expansion does not block additional pixels.

The micro-wells may have a height selected to be between an average height for a microchannel (e.g. -150 um) and a typical assay height (e.g. ~3 mm) for 4 micro-well chip.

To facilitate rapid delivery of different analytes, the device may comprise a plurality of inlet ports for directing a fluid sample into a respective micro-well. A pitch of the inlet ports may be matched to a pitch of multichannel micro-pipette to enable simultaneous delivery. To maintain the reagent fluidic volume and for easy passage of reagents by capillary forces, each micro-well may comprise an outlet. The inlet into each micro-well may include both a capillary conduit to enable liquid delivery through capillary action, as well as one or more reservoirs for reagent mixing before delivery to the micro-well.

In one example, a reference electrode for the micro-wells may be formed on or integrated with the walls that define the micro-wells. For example a 100 um diameter Ag/AgCl electrode may be integrated into the micro-wells from the side of the micro-wells. This arrangement can provide an independent reference electrode for each micro-well, which in turn enables the ISFET function of chip to be used simultaneously in each micro-well.

If the chip is equipped with multiple sensing modalities, the number of analytes that can be assayed simultaneously can multiply by the number of micro-wells that are present. For example, in an arrangement with four micro-wells and two independent sensing modalities, one can assay eight analytes simultaneously in real time.

In one example, the delivery of fluid to the device may be controlled through a fluid management algorithm configured to effect sequential delivery of the reagents including any or all of the steps of (i) diluting of the analyte, (ii) introducing supporting reagents for the reaction, (iii) introducing the sample (e.g. human bodily fluid such as blood, serum, urine, etc.), and (iv) introducing the enzyme to initiate the reaction.

In each of the multiplex assay apparatus described above, the chip and chip carrier may be mounted on a printed circuit board (PCB), where the chip is integrated with a microcontroller to provide addressing signals and to acquire output readings from the array of sensors on the chip. The readings may then be transferred wirelessly or via universal serial bus (USB) to a computer based program (e.g. LabVIEW ®) or android based program in which the data may be processed and analysed.

Where an LED is used to perform the multiplex assays, the optical characteristics of the LED and the sensors on the chip must be evaluated prior to carrying out the assay to examine their spectral relationship. The LED should preferably be selected to emit light having a wavelength which is close to the peak sensitivity of the photosensitive sensors.

Accordingly, the exemplary embodiments of the invention set forth above are considered to be illustrative and not limiting.

REFERENCES

[1] Al-Rawhani, M. A. et al.: A Colorimetric CMOS-Based Platform for Rapid Total Serum Cholesterol Quantification, IEEE SENSORS JOURNAL, 17(2): 240-247

[2] Cheah, B. C. et al.: An Integrated Circuit for Chip-Based Analysis of Enzyme Kinetics and Metabolite Quantification, IEEE Transactions on Biomedical Circuits and Systems 10(3): 721-730

**Claims**

1. An apparatus for detecting biomarkers in a biological sample, the apparatus comprising:

a sample receiving module arranged to receive the biological sample (105), the sample receiving module comprising a capillary transport structure configured to transport the biological sample to a reaction zone, positioned on either the capillary transport structure or on the surface of a CMOS-based sensor unit, for testing, wherein the reaction zone comprises a first testing region and a second testing region spatially separated from the first testing region, wherein properties of the first testing region and the second testing region are affected by the presence of biomarkers to be detected, the first testing region being sensitive to a first biomarker and the second testing region being sensitive to a second biomarker that is different from the first biomarker; and
wherein the CMOS-based sensor unit (102) is adapted to detect independently the properties of the first testing region and the second testing region, wherein the CMOS-based sensor unit is configured to generate separate electrical signals for each of the first testing region and the second testing region, and wherein a change over time in amplitude of the separate electrical signals is indicative of the presence of the first biomarker and second biomarker respectively, wherein the first testing region and the second testing region each comprise a microfluidic channel formed on either the capillary transport structure or a surface of the CMOS-based sensor unit, wherein the microfluidic channel of the first testing region is activated to respond to the first biomarker and the microfluidic channel of the second testing region is activated to respond to the second biomarker,

wherein, when the microfluidic channels are formed on the capillary transport structure, the capillary transport structure is configured to not contact the surface of the CMOS-based sensor unit, and

wherein, when the microfluidic channels are formed on the surface of the CMOS-based sensor unit, the capillary transport structure includes corresponding microfluidic channels configured to contact a surface of the CMOS-based sensor unit.

2. An apparatus according to claim 1, wherein the capillary transport structure comprises a paper strip (104) disposed over the CMOS-based sensor unit, and wherein the first testing region and the second testing region are integrally formed in the paper strip.

3. An apparatus according to claim 1, wherein the reaction zone comprises microfluidic channels (110, 111, 112) arranged to draw the biological sample to the first testing region and the second testing region, which are disposed on the CMOS-based sensor unit.

4. An apparatus according to any preceding claim comprising a fluid flow barrier separating the first testing region from the second testing region.

5. An apparatus (600) for detecting biomarkers in a biological sample, the apparatus comprising:

a sample receiving module arranged to receive the biological sample and transport it to a reaction zone for testing, wherein the reaction zone comprises a first testing region (605a) and a second testing region (605b) spatially separated from the first testing region, wherein properties of the first testing region and the second testing region are configured to be independently affected by the presence of a biomarker to be detected; and

a CMOS-based sensor unit (601) disposed in relation to the reaction zone to detect independently the properties of the first testing region and the second testing region thereby to generate separate signals indicative of the presence of the biomarker to be detected in the first testing region and the second testing region respectively,

wherein the reaction zone comprises a base layer and a separation wall upstanding from the base layer to define separate reaction chambers corresponding to the first testing region and second testing region,

wherein the base layer and separation wall are formed from epoxy,

wherein the first testing region and the second testing region each comprise a respective micro-well formed by an absence of the base layer over a respective portion of the CMOS-based sensor unit, the micro-wells being separated from each other by a barrier portion (603) formed by the separation wall contacting a surface of the CMOS-based sensor unit.

6. An apparatus according to claim 5, wherein the barrier portion is a wall formed from black epoxy.

7. An apparatus according to any preceding claim, wherein the first testing region is sensitive to a first biomarker, and the second testing region is sensitive to a second biomarker, whereby the separate signals are indicative of the presence of the first biomarker and second biomarker respectively.

8. An apparatus according to any preceding claim, wherein the reaction zone includes a control region that is not sensitive to the presence of the biomarker to be detected.

9. An apparatus according to any preceding claim, wherein the CMOS-based sensor unit comprises an optical sensor.

10. An apparatus according to any preceding claim, wherein the CMOS-based sensor unit comprises a substrate having fabricated thereon:

a first sensing element configured to provide a first sensing modality; and
a second sensing element configured to provide a second sensing modality.

11. An apparatus according to claim 10, wherein the first sensing element comprises an optical sensor that incorporates a photodiode and/or single photon avalanche diode, and wherein the second sensing element is a chemical sensor.

12. An apparatus according to claim 11, wherein the second sensing element is a pH sensor comprising an ion sensitive field effect transistor (ISFET) having a gate electrode in contact with the reaction zone.

13. An apparatus according to any preceding claim comprising an array of CMOS-based sensor units, wherein each CMOS-based sensor unit in the array is independently addressable.

**14.** A method of detecting biomarkers in a biological sample, the method comprising:

applying, in a sample receiving module of a detection apparatus, the biological sample to a capillary transport structure;

transporting the biological sample by the capillary transport structure to a reaction zone comprising a first testing region and a second testing region spatially separated from the first testing region, wherein properties of the first testing region and the second testing region are affected by the presence of biomarkers to be detected, the first testing region being sensitive to a first biomarker and the second testing region being sensitive to a second biomarker that is different from the first biomarker;

simultaneously measuring properties of the first testing region and the second testing region using a CMOS-based sensor unit that is disposed in relation to the reaction zone to generate separate electrical signals indicative of the presence of biomarkers in each of the first testing region and the second testing region, the separate electrical signals being indicative of the presence of the first biomarker and second biomarker respectively; and

determining the presence of a plurality of biomarkers from a change over time in amplitude of the separate electrical signals obtained by the CMOS-based sensor unit for the first testing region and the second testing region.

**15.** A method according to claim 14, wherein the CMOS-based sensor unit comprises an optical sensor, and wherein the method includes:

illuminating the reaction zone with optical radiation, or

performing a chemiluminescence assay.

**Patentansprüche**

**1.** Vorrichtung zum Detektieren von Biomarkern in einer biologischen Probe, wobei die Vorrichtung Folgendes umfasst:

ein Probenaufnahmemodul, das angeordnet ist, um die biologische Probe (105) zum Testen aufzunehmen, wobei das Probenaufnahmemodul eine Kapillartransportstruktur umfasst, die ausgelegt ist, um die biologische Probe in eine Reaktionszone zu transportieren, die entweder auf der Kapillartransportstruktur oder auf der Oberfläche einer Sensoreinheit auf CMOS-Basis angeordnet ist, wobei die Reaktionszone einen ersten Testbereich und einen zweiten Testbereich umfasst, der räumlich von dem ersten Testbereich getrennt ist, wobei Eigenschaften des ersten Testbereichs und des zweiten Testbereichs durch das Vorhandensein von zu detektierenden Biomarkern beeinflusst werden, wobei der erste Testbereich gegenüber einem ersten Biomarker empfindlich ist und der zweite Testbereich gegenüber einem zweiten Biomarker empfindlich ist, der sich von dem ersten Biomarker unterscheidet; und

wobei die Sensoreinheit (102) auf CMOS-Basis ausgelegt ist, um unabhängig die Eigenschaften des ersten Testbereichs und des zweiten Testbereichs zu detektieren, wobei die Sensoreinheit auf CMOS-Basis ausgelegt ist, um separate elektrische Signale für jeden aus dem ersten Testbereich und dem zweiten Testbereich zu erzeugen, und wobei eine Änderung über die Zeit der Amplitude der separaten elektrischen Signale das Vorhandensein des ersten Biomarkers bzw. des zweiten Biomarkers angibt,

wobei der erste Testbereich und der zweite Testbereich jeweils einen Mikrofluidkanal umfassen, der entweder auf der Kapillartransportstruktur oder auf einer Oberfläche der Sensoreinheit auf CMOS-Basis ausgebildet ist, wobei der Mikrofluidkanal des ersten Testbereichs aktiviert wird, um auf den ersten Biomarker zu reagieren, und der Mikrofluidkanal des zweiten Testbereichs aktiviert wird, um auf den zweiten Biomarker zu reagieren,

wobei, wenn die Mikrofluidkanäle auf der Kapillartransportstruktur ausgebildet sind, die Kapillartransportstruktur ausgelegt ist, mit der Oberfläche der Sensoreinheit auf CMOS-Basis nicht in Kontakt zu sein, und

wobei, wenn die Mikrofluidkanäle auf der Oberfläche der Sensoreinheit auf CMOS-Basis ausgebildet sind, die Kapillartransportstruktur entsprechende Mikrofluidkanäle umfasst, die ausgelegt sind, um mit einer Oberfläche der Sensoreinheit auf CMOS-Basis in Kontakt zu sein.

**2.** Vorrichtung nach Anspruch 1, wobei die Kapillartransportstruktur einen Papierstreifen (104) umfasst, der über der Sensoreinheit auf CMOS-Basis angeordnet ist, und wobei der erste Testbereich und der zweite Testbereich einstückig in dem Papierstreifen ausgebildet sind.

**3.** Vorrichtung nach Anspruch 1, wobei die Reaktionszone Mikrofluidkanäle (110, 111, 112) umfasst, die angeordnet sind, um die biologische Probe in den ersten Testbereich und den zweiten Testbereich zu ziehen, die auf der Sensoreinheit auf CMOS-Basis angeordnet sind.

**4.** Vorrichtung nach einem der vorangegangenen Ansprüche, umfassend eine Fluidströmungsbarriere, die den ersten Testbereich vom zweiten Testbereich trennt.

5. Vorrichtung (600) zum Detektieren von Biomarkern in einer biologischen Probe, wobei die Vorrichtung Folgendes umfasst:

ein Probeaufnahmemodul, das angeordnet ist, um die biologische Probe aufzunehmen und sie zum Testen in eine Reaktionszone zu transportieren, wobei die Reaktionszone einen ersten Testbereich (605a) und einen zweiten Testbereich (605b) umfasst, der räumlich von dem ersten Testbereich getrennt ist, wobei Eigenschaften des ersten Testbereichs und des zweiten Testbereichs ausgelegt sind, um unabhängig vom Vorhandensein eines zu detektierenden Biomarkers beeinflusst zu werden; und
eine Sensoreinheit auf CMOS-Basis (601), die in Verbindung mit der Reaktionszone angeordnet ist, um unabhängig die Eigenschaften des ersten Testbereichs und des zweiten Testbereichs zu detektieren, um dadurch separate Signale zu erzeugen, die das Vorhandensein des zu detektierenden Biomarkers in dem ersten Testbereich bzw. dem zweiten Testbereich angeben,
wobei die Reaktionszone eine Basisschicht und eine Trennwand umfasst, die ausgehend von der Basisschicht nach oben ragt, um getrennte Reaktionskammern zu definieren, die dem ersten Testbereich und dem zweiten Testbereich entsprechen,
wobei die Basisschicht und die Trennwand aus Epoxy ausgebildet sind,
wobei der erste Testbereich und der zweite Testbereich jeweils ein entsprechendes Mikro-Well umfassen, das durch ein Nichtvorhandensein der Basisschicht über einem entsprechenden Teil der Sensoreinheit auf CMOS-Basis ausgebildet ist, wobei die Mikro-Wells durch einen Barrierenteil (603) voneinander getrennt sind, der durch die Trennwand, die mit einer Oberfläche der Sensoreinheit auf CMOS-Basis in Kontakt ist, ausgebildet ist.

6. Vorrichtung nach Anspruch 5, wobei der Barrierenteil eine Wand ist, die aus schwarzem Epoxy ausgebildet ist.

7. Vorrichtung nach einem der vorangegangenen Ansprüche, wobei der erste Testbereich gegenüber einem ersten Biomarker empfindlich ist und der zweite Testbereich gegenüber einem zweiten Biomarker empfindlich ist, wodurch die separaten Signale das Vorhandensein des ersten Biomarkers bzw. des zweiten Biomarkers angeben.

8. Vorrichtung nach einem der vorangegangenen Ansprüche, wobei die Reaktionszone einen Kontrollbereich umfasst, der nicht gegenüber dem Vorhandensein des zu detektierenden Biomarkers empfindlich ist.

9. Vorrichtung nach einem der vorangegangenen Ansprüche, wobei die Sensoreinheit auf CMOS-Basis einen optischen Sensor umfasst.

10. Vorrichtung nach einem der vorangegangenen Ansprüche, wobei die Sensoreinheit auf CMOS-Basis ein Substrat umfasst, das darauf ausgebildet Folgendes umfasst:

ein erstes Abfühlelement, das ausgelegt ist, um eine erste Abfühlmodalität bereitzustellen; und
ein zweites Abfühlelement, das ausgelegt ist, um eine zweite Abfühlmodalität bereitzustellen.

11. Vorrichtung nach Anspruch 10, wobei das erste Abfühlelement einen optischen Sensor umfasst, der eine Photodiode und/oder Einzelphotonen-Lawinendiode einschließt, und wobei das zweite Abfühlelement ein chemischer Sensor ist.

12. Vorrichtung nach Anspruch 11, wobei das zweite Abfühlelement ein pH-Sensor ist, der einen ionensensitiven Feldeffekttransistor (ISFET) mit einer Gate-Elektrode in Kontakt mit der Reaktionszone umfasst.

13. Vorrichtung nach einem der vorangegangenen Ansprüche, umfassend eine Anordnung von Sensoreinheiten auf CMOS-Basis, wobei jede Sensoreinheit auf CMOS-Basis in der Anordnung unabhängig adressierbar ist.

14. Verfahren zum Detektieren von Biomarkern in einer biologischen Probe, wobei das Verfahren Folgendes umfasst:

Aufbringen der biologischen Probe auf eine Kapillartransportstruktur in einem Probeaufnahmemodul einer Detektionsvorrichtung;
Transportieren der biologischen Probe durch die Kapillartransportstruktur an eine Reaktionszone, umfassend einen ersten Testbereich und einen zweiten Testbereich, der räumlich von dem ersten Testbereich getrennt ist, wobei Eigenschaften des ersten Testbereichs und des zweiten Testbereichs durch das Vorhandensein von zu detektierenden Biomarkern beeinflusst werden, wobei der erste Testbereich gegenüber einem ersten Biomarker empfindlich ist und der zweite Testbereich gegenüber einem zweiten Biomarker empfindlich ist, der sich von dem ersten Biomarker unterscheidet;
gleichzeitiges Messen von Eigenschaften des ersten Testbereichs und des zweiten Testbereichs unter Verwendung einer Sensoreinheit auf CMOS-Basis, die in Verbindung mit der Reaktionszone bereitgestellt ist, um separate elektrische Signale zu erzeugen, die das Vorhandensein von Biomarkern in jedem aus dem ersten Testbereich und dem zweiten Testbereich angeben, wobei die separaten elektrischen Signale das Vorhandensein des ersten Biomarkers bzw. des zweiten Biomarkers angeben; und

Bestimmen des Vorhandenseins einer Vielzahl von Biomarkern aus einer Änderung über der Zeit der Amplitude der separaten elektrischen Signale, die durch die Sensoreinheit auf CMOS-Basis erhalten wurden, für den ersten Testbereich und den zweiten Testbereich.

15. Verfahren nach Anspruch 14, wobei die Sensoreinheit auf CMOS-Basis einen optischen Sensor umfasst, und wobei das Verfahren Folgendes umfasst:

Bestrahlen der Reaktionszone mit optischer Strahlung oder
Durchführen eines Chemilumineszenzassays.


**Revendications**

1. Appareil pour détecter des biomarqueurs dans un échantillon biologique, l'appareil comprenant :

un module de réception d'échantillon agencé pour recevoir l'échantillon biologique (105), le module de réception d'échantillon comprenant une structure de transport capillaire configurée pour transporter l'échantillon biologique vers une zone de réaction, positionnée soit sur la structure de transport capillaire soit sur la surface d'une unité de capteur à base de CMOS, pour un test, dans lequel la zone de réaction comprend une première région de test et une seconde région de test séparée spatialement de la première région de test, dans lequel des propriétés de la première région de test et de la seconde région de test sont affectées par la présence de biomarqueurs à détecter, la première région de test étant sensible à un premier biomarqueur et la seconde région de test étant sensible à un second biomarqueur qui est différent du premier biomarqueur ; et
dans lequel l'unité de capteur à base de CMOS (102) est adaptée pour détecter indépendamment les propriétés de la première région de test et de la seconde région de test, dans lequel l'unité de capteur à base de CMOS est configurée pour générer des signaux électriques séparés pour chacune de la première région de test et du second test, dans lequel un changement au fil du temps de l'amplitude des signaux électriques séparés indique la présence du premier biomarqueur et du second biomarqueur, respectivement,
dans lequel la première région de test et la seconde région de test comprennent chacune un canal microfluidique formé soit sur la structure de transport capillaire soit sur une surface de l'unité de capteur à base de CMOS, dans lequel le canal microfluidique de la première région de test est activé pour répondre au premier biomarqueur et le canal microfluidique de la seconde région de test est activé pour répondre au second biomarqueur,
dans lequel, lorsque les canaux microfluidiques sont formés sur la structure de transport capillaire, la structure de transport capillaire est configurée pour ne pas venir en contact avec la surface de l'unité de capteur à base de CMOS, et
dans lequel, lorsque les canaux microfluidiques sont formés sur la surface de l'unité de capteur à base de CMOS, la structure de transport capillaire comprend des canaux microfluidiques correspondants configurés pour venir en contact avec une surface de l'unité de capteur à base de CMOS.

2. Appareil selon la revendication 1, dans lequel la structure de transport capillaire comprend une bande de papier (104) disposée au-dessus de l'unité de capteur à base de CMOS, et dans lequel la première région de test et la seconde région de test sont formées d'un seul tenant dans la bande de papier.

3. Appareil selon la revendication 1, dans lequel la zone de réaction comprend des canaux microfluidiques (110, 111, 112) agencés pour attirer l'échantillon biologique vers la première région de test et la seconde région de test, qui sont disposés sur l'unité de capteur à base de CMOS.

4. Appareil selon l'une quelconque des revendications précédentes, comprenant une barrière à un écoulement de fluide séparant la première région de test de la seconde région de test.

5. Appareil (600) pour détecter des biomarqueurs dans un échantillon biologique, l'appareil comprenant :

un module de réception d'échantillon agencé pour recevoir l'échantillon biologique et le transporter vers une zone de réaction pour un test, dans lequel la zone de réaction comprend une première région de test (605a) et une seconde région de test (605b) séparées spatialement de la première région de test, dans lequel des propriétés de la première région de test et de la

14

seconde région de test sont configurées pour être affectées indépendamment par la présence d'un biomarqueur à détecter ; et

une unité de capteur à base de CMOS (601) disposée par rapport à la zone de réaction pour détecter indépendamment les propriétés de la première région de test et de la seconde région de test afin de générer ainsi des signaux distincts indicatifs de la présence du biomarqueur à détecter dans la première région de test et la seconde région de test, respectivement,

dans lequel la zone de réaction comprend une couche de base et une paroi de séparation s'élevant à partir de la couche de base pour définir des chambres de réaction séparées correspondant à la première région de test et à la seconde région de test,

dans lequel la couche de base et la paroi de séparation sont formées à partir d'époxy,

dans lequel la première région de test et la seconde région de test comprennent chacune un micro-puits respectif formé par une absence de la couche de base sur une partie respective de l'unité de capteur à base de CMOS, les micro-puits étant séparés les uns des autres par une partie de barrière (603) formée par la paroi de séparation venant en contact avec une surface de l'unité de capteur à base de CMOS.

6. Appareil selon la revendication 5, dans lequel la partie barrière est une paroi formée d'époxy noir.

7. Appareil selon l'une quelconque des revendications précédentes, dans lequel la première région de test est sensible à un premier biomarqueur, et la seconde région de test est sensible à un second biomarqueur, moyennant quoi les signaux séparés sont indicatifs de la présence du premier biomarqueur et du second biomarqueur, respectivement.

8. Appareil selon l'une quelconque des revendications précédentes, dans lequel la zone de réaction comprend une région de commande qui n'est pas sensible à la présence du biomarqueur à détecter.

9. Appareil selon l'une quelconque des revendications précédentes, dans lequel l'unité de capteur à base de CMOS comprend un capteur optique.

10. Appareil selon l'une quelconque des revendications précédentes, dans lequel l'unité de capteur à base de CMOS comprend un substrat sur lequel est fabriqué :

un premier élément de détection configuré pour fournir une première modalité de détection ; et un second élément de détection configuré pour fournir une seconde modalité de détection.

11. Appareil selon la revendication 10, dans lequel le premier élément de détection comprend un capteur optique qui incorpore une photodiode et/ou une diode à avalanche à photon unique, et dans lequel le second élément de détection est un capteur chimique.

12. Appareil selon la revendication 11, dans lequel le second élément de détection est un capteur de pH comprenant un transistor à effet de champ sensible aux ions (ISFET) présentant une électrode de grille en contact avec la zone de réaction.

13. Appareil selon l'une quelconque des revendications précédentes, comprenant un réseau d'unités de capteur à base de CMOS, dans lequel chaque unité de capteur à base de CMOS dans le réseau est adressable indépendamment.

14. Procédé de détection de biomarqueurs dans un échantillon biologique, le procédé comprenant les étapes consistant à :

appliquer, dans un module de réception d'échantillon d'un appareil de détection, l'échantillon biologique à une structure de transport capillaire ;

transporter l'échantillon biologique par l'intermédiaire de la structure de transport capillaire vers une zone de réaction comprenant une première région de test et une seconde région de test séparée spatialement de la première région de test, dans lequel des propriétés de la première région de test et de la seconde région de test sont affectées par la présence de biomarqueurs à détecter, la première région de test étant sensible à un premier biomarqueur et la seconde région de test étant sensible à un second biomarqueur qui est différent du premier biomarqueur ;

mesurer simultanément des propriétés de la première région de test et de la seconde région de test à l'aide d'une unité de capteur à base de CMOS qui est disposée par rapport à la zone de réaction pour générer des signaux électriques séparés indiquant la présence de biomarqueurs dans chacune de la première région de test et de la seconde région de test, les signaux électriques distincts indiquant la présence du premier biomarqueur et du second biomarqueur, respectivement ; et

déterminer la présence d'une pluralité de biomarqueurs à partir d'un changement dans le temps de l'amplitude des signaux électriques séparés obtenus par l'unité de capteur à base de CMOS pour la première région de test et la seconde région de test.

**15.** Procédé selon la revendication 14, dans lequel l'unité de capteur à base de CMOS comprend un capteur optique, et dans lequel le procédé comprend les étapes consistant à :

éclairer la zone de réaction avec un rayonnement optique, ou
effectuer un test de chimiluminescence.

1001 1002

FIG. 1

106 105
104
103
101
102

FIG. 2

FIG. 3

FIG. 4

FIG. 5

FIG. 6

FIG. 7

FIG. 8

406

404

413

415

414

FIG. 9

500

501

502

503

504

FIG. 10

FIG. 11

FIG. 12

FIG. 13

570

571

573
578
572
574
576
575

FIG. 14

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20160080548 A
- WO 2010047804 A
- US 20050249633 A1
- US 20130161190 A1
- EP 2230314 A1

**Non-patent literature cited in the description**

- **AL-RAWHANI, M. A. et al.** A Colorimetric CMOS-Based Platform for Rapid Total Serum Cholesterol Quantification. *IEEE SENSORS JOURNAL,* vol. 17 (2), 240-247
- **CHEAH, B. C. et al.** An Integrated Circuit for Chip-Based Analysis of Enzyme Kinetics and Metabolite Quantification. *IEEE Transactions on Biomedical Circuits and Systems,* vol. 10 (3), 721-730